(19) Europäisches Patentamt / European Patent Office / Office européen des brevets

(11) **EP 3 995 917 B1**

(12) **EUROPEAN PATENT SPECIFICATION**

(45) Date of publication and mention
of the grant of the patent:
**09.07.2025  Bulletin 2025/28**

(21) Application number: **20834542.1**

(22) Date of filing: **19.05.2020**

(51) International Patent Classification (IPC):
**G05B 19/418** (2006.01)   **G05B 11/36** (2006.01)
**G05B 13/02** (2006.01)   **B01J 19/00** (2006.01)
**G16C 20/10** (2019.01)   **G16C 20/70** (2019.01)
**G06N 3/04** (2023.01)   **G06N 3/08** (2023.01)

(52) Cooperative Patent Classification (CPC):
**G05B 19/41875; B01J 19/0006; B01J 19/0033;
B01J 19/004; G06N 3/08; G06N 20/20;**
B01J 2219/00033; B01J 2219/00193;
B01J 2219/00227; G05B 2219/32187;
G05B 2219/32188; G05B 2219/32193;
G05B 2219/32194; G06N 7/01; G16C 20/10;
(Cont.)

(86) International application number:
**PCT/JP2020/019773**

(87) International publication number:
**WO 2021/002108 (07.01.2021 Gazette 2021/01)**

(54) **OPTIMIZATION SUPPORT DEVICE, METHOD, AND PROGRAM**

OPTIMIERUNGSSTÜTZVORRICHTUNG, VERFAHREN UND PROGRAMM

DISPOSITIF, PROCÉDÉ ET PROGRAMME DE SUPPORT D'OPTIMISATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **03.07.2019  JP 2019124421**

(43) Date of publication of application:
**11.05.2022  Bulletin 2022/19**

(73) Proprietor: **FUJIFILM Corporation
Tokyo 106-8620 (JP)**

(72) Inventor: **HASEGAWA, Masataka
Minamiashigara-shi, Kanagawa 250-0193 (JP)**

(74) Representative: **Meissner Bolte Partnerschaft
mbB
Patentanwälte Rechtsanwälte
Postfach 86 06 24
81633 München (DE)**

(56) References cited:
WO-A1-2019/106963    WO-A1-99/00717
WO-A2-2018/072773    JP-A- 2011 194 728
JP-A- 2015 172 025    JP-A- 2015 223 175
JP-A- 2015 223 550    JP-A- H06 304 724
US-A1- 2008 300 709

• YU JIE ET AL: "A Bayesian model averaging
based multi-kernel Gaussian process regression
framework for nonlinear state estimation and
quality prediction of multiphase batch processes
with transient dynamics and uncertainty",
CHEMICAL ENGINEERING SCIENCE, OXFORD,
GB, vol. 93, 13 February 2013 (2013-02-13), pages
96 - 109, XP028525786, ISSN: 0009-2509, DOI:
10.1016/J.CES.2013.01.058

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- YU JIE ET AL: "Multi-kernel Gaussian process regression and Bayesian model averaging based nonlinear state estimation and quality prediction of multiphase batch processes", 2015 AMERICAN CONTROL CONFERENCE (ACC), IEEE, 17 June 2013 (2013-06-17), pages 5451 - 5456, XP032476062, ISSN: 0743-1619, [retrieved on 20130814], DOI: 10.1109/ACC.2013.6580690
- YINGWEI ZHANG ET AL: "Optimization of nonlinear process based on sequential extreme learning machine", CHEMICAL ENGINEERING SCIENCE, OXFORD, GB, vol. 66, no. 20, 15 June 2011 (2011-06-15), pages 4702 - 4710, XP028266287, ISSN: 0009-2509, [retrieved on 20110623], DOI: 10.1016/J.CES.2011.06.030

(52) Cooperative Patent Classification (CPC): (Cont.)
G16C 20/70; Y02P 90/02

**Description**

**BACKGROUND OF THE INVENTION**

1. Field of the Invention

The present disclosure relates to a production facility comprising a flow reactor and a support device that supports optimization of production process parameters.

2. Description of the Related Art

In the process of producing a product, for example, a method of associating a process condition parameter that specifies operating conditions and the like of the process with a quality parameter of the product and predicting the quality parameter as a forward problem from the process condition parameter or predicting an optimum solution of the process condition parameter by an inverse problem from the quality parameter has been proposed by using various operations using a neural network.

For example, JP2003-345416A proposes a method of deriving an optimum solution of a production plan in the reverse order of the flow of producing a product from a raw material in order to divide a product production process, define a plurality of prediction models, and optimize a product production plan. The method described in JP2003-345416A corresponds to a method of predicting a process condition parameter by an inverse problem from a quality parameter of a product by learning the correlation between the process condition parameter and the quality parameter. In addition, JP2006-323523A proposes a method of modeling a production process by operating variables, state variables, and quality variables, and deriving optimum operating variables in order to improve the quality of the product. The method described in JP2006-323523A corresponds to a method of predicting a quality parameter as a forward problem from a process condition parameter.

JP H06 304724 A (NIPPON STEEL CORP) 1 November 1994 (1994-11-01) discloses a system controlling quality defects such as wrinkles in steel manufacturing.WO 2018/072773 A2 (REIFENHAUSER GMBH & CO. KG MASCHINENFABRIK) discloses a system producing extrusion products wherein the control uses machine learning to determine dependence between parameters.WO 99/00717 A1 (HONEYWELL INC [US]) 7 January 1999 (1999-01-07) discloses a flow reactor with closed-loop control based on product quality.

**SUMMARY OF THE INVENTION**

Incidentally, in the process of producing a product, there are a considerable number of process condition parameters that specify the operating conditions and the like of the process. In a case where many such condition parameters are input to the prediction model to predict the quality parameters of the product, accurate prediction may not be possible. In particular, in a case where the prediction model is a neural network, a large number of process condition parameters results in overtraining of the neural network. In a case where the neural network is over-trained in this way, accurate quality parameters cannot be predicted in a case where process condition parameters other than teacher data are input. It is also conceivable to use a function or table instead of the prediction model to obtain quality parameters from process condition parameters. However, even in such cases, a large number of process condition parameters complicates the configuration of the function or table, making it difficult to create an appropriate function or table, and as a result, accurate prediction may not be possible.

The present invention has been made in view of the above circumstances, and an object of the present invention is to enable accurate prediction of various conditions in a product production process.

The invention is defined by claim 1.

**BRIEF DESCRIPTION OF THE DRAWINGS**

Fig. 1 is a schematic block diagram showing a configuration of a production facility to which an optimization support device according to an embodiment is applied.
Fig. 2 is a schematic block diagram showing a configuration of a production facility including a flow reactor.
Fig. 3 is a schematic block diagram showing a configuration of the optimization support device according to the present embodiment.
Fig. 4 is a conceptual diagram of a layer structure of a neural network.
Fig. 5 is a conceptual diagram of a layer structure of a neural network.

Fig. 6 is an explanatory diagram of a data set of teacher data.

Fig. 7 is a conceptual diagram of processing performed in the present embodiment.

Fig. 8 is a flowchart showing a process performed at the time of generating a first learning model of a first conversion unit and a second learning model of a second conversion unit in the present embodiment.

Fig. 9 is a flowchart showing a process for deriving an operating condition parameter from a target quality parameter.

Fig. 10 is a schematic block diagram showing a configuration of an optimization support device according to another embodiment.

Fig. 11 is a schematic block diagram showing a configuration of an optimization support device according to still another embodiment.

## DESCRIPTION OF THE PREFERRED EMBODIMENTS

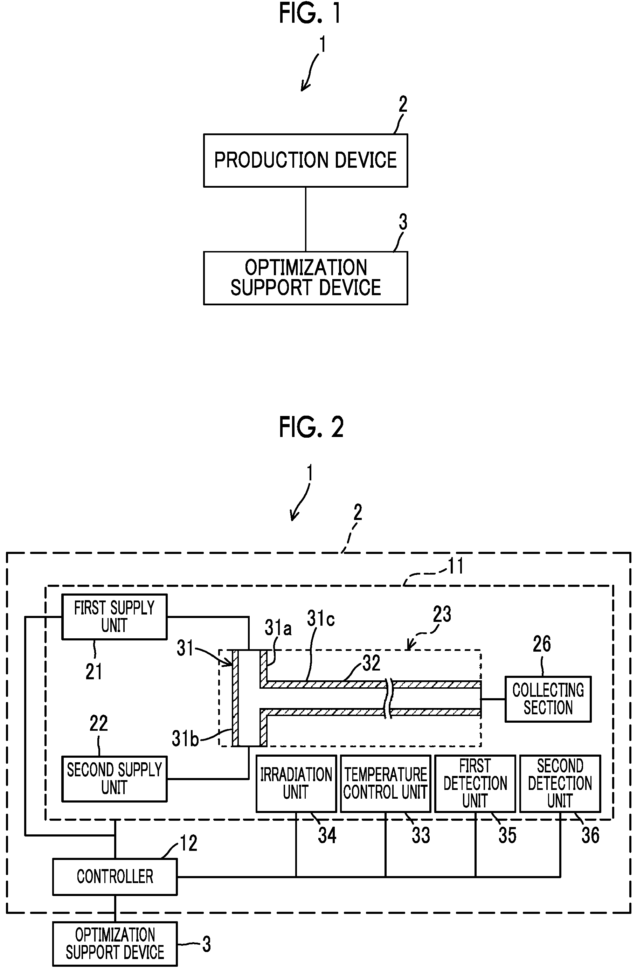

Hereinafter, an embodiment of the present disclosure will be described with reference to the drawings. Fig. 1 is a schematic block diagram showing a configuration of a production facility to which an optimization support device according to an embodiment of the present disclosure is applied. As shown in Fig. 1, a production facility 1 according to the present embodiment comprises a production device 2 and an optimization support device 3 according to the present embodiment.

In the present embodiment, a flow reactor is included as the production device 2. The flow reactor is a device for obtaining a product by performing a flow reaction process in which a raw material is continuously reacted while flowing. Fig. 2 is a schematic block diagram showing a configuration of the production facility 1 including a flow reactor. As shown in Fig. 2, the production device 2 comprises a flow reactor 11 and a controller 12. The flow reactor 11 comprises a first supply unit 21, a second supply unit 22, a reaction section 23, and a collecting section 26. The operation of each part of the flow reactor 11 is controlled by the controller 12. The controller 12 is connected to the optimization support device 3.

In the flow reactor 11, the first supply unit 21 and the second supply unit 22 are respectively connected to upstream end parts of the reaction section 23 by piping, and the collecting section 26 is connected to a downstream end part of the reaction section 23 by piping.

The flow reaction performed in the flow reactor 11 may be, for example, a synthesis reaction for synthesizing a compound that is a monomer, or a polymerization reaction for producing a polymer by reacting monomers, or may be elementary reactions such as an initiation reaction and a termination reaction in an anionic polymerization reaction, for example. Accordingly, a reactant that is a target of the flow reaction may be, for example, a vegetation (growth) stage compound that is a target of the termination reaction. In the present embodiment, the termination reaction of stopping the vegetation (growth) of polystyryllithium with methanol is performed by the flow reaction.

The first supply unit 21 is a member for supplying a first raw material of the flow reaction to the reaction section 23. The first raw material in the present embodiment is, for example, a first liquid obtained by dissolving polystyryllithium in a solvent, and polystyryllithium is an example of a reactant of the flow reaction process. The first supply unit 21 comprises a pump (not shown), and a flow rate of the first raw material to the reaction section 23 is controlled by controlling a rotation speed of the pump.

The second supply unit 22 is a member for supplying a second raw material of the flow reaction to the reaction section 23. The second raw material in the present embodiment is a mixture of methanol and water, that is, an aqueous methanol solution, and methanol is used as a terminating agent for the termination reaction. The second supply unit 22 also comprises a pump (not shown) like the first supply unit 21, and a flow rate of the second raw material to the reaction section 23 is controlled by controlling a rotation speed of the pump.

The reaction section 23 is a member for performing a termination reaction as a flow reaction, and comprises a merging portion 31, a reaction portion 32, a temperature control unit 33, an irradiation unit 34, and a first detection unit 35. The merging portion 31 is a T-shaped branched tube, that is, a T-shaped tube. A cross tube may be used instead of the T-shaped tube. A first tube part 31a of the merging portion 31 is connected to the first supply unit 21, a second tube part 31b thereof is connected to the second supply unit 22, and a third tube part 31c thereof is connected to the reaction portion 32, respectively. Thus, the first raw material and second raw material guided to the reaction section 23 are combined with each other and are sent to the reaction portion 32 in a mixed state. The reaction portion 32 has a predetermined reaction path length and reaction path diameter. The reaction path length and reaction path diameter can be changed by changing the tubular member constituting the reaction portion 32.

The inside of the reaction portion 32 is a flow path for a mixture (hereinafter referred to as a mixed raw material) of the first raw material and the second raw material, and a hollow portion in the tube is defined as a reaction site. The mixed raw material undergoes an anionic polymerization termination reaction while passing through the reaction portion 32, so that polystyrene is produced.

The temperature control unit 33 consists of, for example, a heater or the like, and is a member for controlling a temperature of the flow reaction (hereinafter referred to as a reaction temperature). The temperature control unit 33 controls the temperature (reaction temperature) of the mixed raw material flowing in and through the merging portion 31 and the reaction portion 32.

The irradiation unit 34 has, for example, a light source that emits light such as ultraviolet rays, and is a member for irradiating the reaction portion 32 with light such as ultraviolet rays in a case of performing a photoreaction as a flow reaction.

The first detection unit 35 detects the state of the mixed raw material in the reaction section 23 and outputs the result to the optimization support device 3. A parameter indicating the state of the mixed raw material (hereinafter referred to as a state parameter) is a parameter indicating the physical properties of the mixed raw material obtained in a case where the mixed raw material is reacted according to the input operating condition parameter and the environment in the reaction section. The state parameter includes, for example, at least one of the reaction temperature, color, pH, and dissolved oxygen content of the mixed raw material, pressure of reaction section 23, or the shape of a spectrum (infrared absorption spectrum, Raman spectroscopic waveform, and nuclear magnetic resonance waveform) representing the physical characteristics of the product. For this purpose, the first detection unit 35 comprises a temperature sensor, an imaging unit, a pH sensor, a dissolved oxygen content sensor, a spectrometer, and the like (all not shown).

The collecting section 26 is a member for collecting polystyrene that is a product of the flow reaction. The collecting section 26 precipitates polystyrene from the polystyrene solution guided from the reaction section, collects the precipitated polystyrene from the mixed solution, and dries the collected polystyrene to obtain polystyrene.

In addition, the collecting section 26 comprises a second detection unit 36. The second detection unit 36 detects the quality of the product, which is the processing result of the flow reaction, and outputs the detected result to the optimization support device 3. A parameter indicating the quality of the product (hereinafter referred to as a quality parameter) is a parameter that serves as a measure for determining whether or not the product obtained as a result of the reaction has appropriate quality. Specifically, the quality parameter includes at least one of the product concentration, the impurity concentration, or the like, but in addition to these, at least one of the product purity, molecular weight, molecular weight dispersion, yield, or the like may be used. In addition, in a case where the product is obtained in the collecting section 26 in a solution state in which the product is dissolved in a solvent, for example, the concentration of the product in the solution (molar concentration or the like) may be detected as a quality parameter.

Meanwhile, the reaction section and the collecting section are not limited to the above examples, and may be appropriately changed depending on at least one of the type of the flow reaction or the type of the product. For example, a container may be provided in place of the collecting section 26, and the polystyrene solution guided from the reaction section 23 may be temporarily stored in this container. In this case, for example, the stored polystyrene solution is guided to the collecting section 26, and the product may be obtained by precipitating, collecting, and drying the polystyrene.

The controller 12 generally controls the flow reactor 11. The controller 12 is connected to each of the pumps of the first supply unit 21 and the second supply unit 22, the temperature control unit 33, the irradiation unit 34, the first detection unit 35, and the second detection unit 36. The controller 12 controls the respective flow rates of the first raw material and the second raw material by respectively controlling the rotation speeds of the pumps of the first supply unit 21 and the second supply unit 22. Further, the controller 12 controls the temperature of the mixed raw material by controlling the temperature control unit 33. Further, the controller 12 controls the irradiation of the reaction section 23 with light such as ultraviolet rays by giving an instruction to the irradiation unit 34. Further, the controller 12 detects the state parameter and the quality parameter by giving an instruction to the first detection unit 35 and the second detection unit 36.

The controller 12 also sets the operating conditions of the flow reactor 11. The parameter indicating the operating condition (hereinafter referred to as the operating condition parameter) is a parameter for setting the reaction condition which is the processing condition of the flow reaction process, and for driving each part of the flow reactor 11 in order to produce a product of an appropriate quality. The operating condition parameter includes, for example, at least one of the flow rate of the first raw material, the flow rate of the second raw material, the reaction time, the reaction temperature, the mixing ratio, the UV illuminance, the flow path depth, or the reagent equivalent in a case where the reagent. The controller 12 has an operating unit (not shown), and sets an operating condition parameter by inputting an operating signal from the operating unit, thereby controlling the flow reactor 11 to the set operating condition. For example, the operating condition parameter is set by click or selection using a mouse in the operating unit and/or input of characters using a keyboard.

Further, the controller 12 is connected to the optimization support device 3, and in addition to or instead of the operating signal from the above-mentioned operating unit, the controller 12 sets the operating condition to a target operating condition output by the optimization support device 3, thereby controlling the flow reactor 11 to the predetermined operating condition.

The optimization support device 3 provides support for accurately determining a target operating condition parameter for the flow reaction process performed by the flow reactor 11. The optimization support device 3 includes, for example, one computer in which an optimization support program according to the present embodiment is installed. The optimization support program is stored in a storage device of a server computer connected to a network or in a network storage in a state in which it can be accessed from the outside, and is downloaded and installed on the computer in response to a request of an operator. Alternatively, the optimization support program is recorded on a recording medium, such as a digital versatile disc (DVD) or a compact disc read only memory (CD-ROM), and distributed, and installed on the computer from the recording medium.

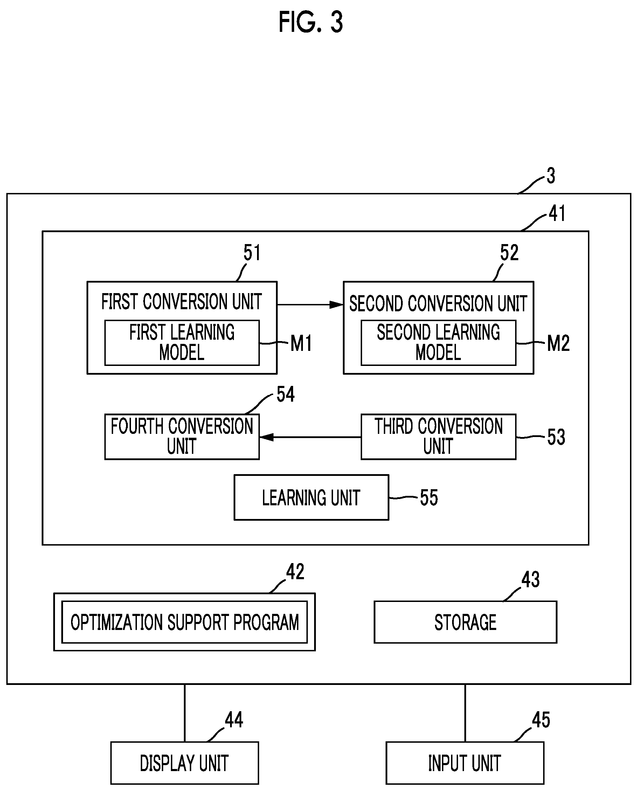

Fig. 3 is a schematic block diagram showing a configuration of the optimization support device realized by installing an optimization support program on a computer. As shown in Fig. 3, the optimization support device 3 comprises a central processing unit (CPU) 41, a memory 42, and a storage 43 as the configuration of a standard computer. Further, the optimization support device 3 is connected to a display unit 44 such as a liquid crystal display and an input unit 45 such as a keyboard and a mouse.

The storage 43 consists of a hard disk drive or the like, and stores various information including information necessary for processing of optimization support.

Further, the memory 42 stores an optimization support program. The optimization support program defines, as processes to be executed by the CPU 41, a first conversion process for converting an operating condition parameter indicating a process operating condition for producing a product into a state parameter indicating a state of the process, a second conversion process for converting the state parameter into a quality parameter indicating a quality of the product, a third conversion process for converting the quality parameter into the state parameter, a fourth conversion process for converting the state parameter into the operating condition parameter, and a learning process for learning a neural network, which will be described later.

Then, in a case where the CPU 41 executes these processes according to the program, the computer functions as a first conversion unit 51, a second conversion unit 52, a third conversion unit 53, a fourth conversion unit 54, and a learning unit 55.

The first conversion unit 51 converts an operating condition parameter indicating an operating condition of a process for producing a product (flow reaction in the present embodiment) into a state parameter indicating a state of the process, thereby deriving the state parameter. For this purpose, the first conversion unit 51 has a first learning model M1 that has been trained to output a state parameter by inputting an operating condition parameter. The first learning model M1 is constructed by the learning unit 55 learning a neural network or the like as described later.

The second conversion unit 52 converts the state parameter into a quality parameter indicating the quality of the product (polystyrene in the present embodiment), thereby deriving the quality parameter. For this purpose, the second conversion unit 52 has a second learning model M2 that has been trained to output a quality parameter by inputting a state parameter. The second learning model M2 is constructed by the learning unit 55 learning a neural network or the like as described later.

The third conversion unit 53 converts the quality parameter indicating the quality of the product into the state parameter indicating the state of the process, thereby deriving the state parameter. In the present embodiment, the third conversion unit 53 converts the quality parameter into the state parameter based on the control parameter of the second learning model M2 in the second conversion unit 52.

The fourth conversion unit 54 converts the state parameter indicating the process state into an operating condition parameter indicating the operating condition of the process, thereby deriving the operating condition parameter. In the present embodiment, the fourth conversion unit 54 converts the state parameter into the operating condition parameter based on the control parameter of the first learning model M1 in the first conversion unit 51.

Here, the model used for the first learning model M1 is a prediction model that predicts a state parameter by inputting an operating condition parameter. The model used for the second learning model M2 is also a prediction model that predicts a quality parameter by inputting a state parameter. A machine learning model can be used as the prediction model. Examples of the machine learning model include linear regression, Gaussian process regression, support vector regression, a decision tree, an ensemble method, a bagging method, a boosting method, a gradient boosting method, and the like. Further, as an example of the machine learning model, a neural network model can be mentioned. Examples of the neural network model include a simple perceptron, a multilayer perceptron, a deep neural network, a convolutional neural network, a deep belief network, a recurrent neural network, a stochastic neural network, and the like.

Random forest can be mentioned as an ensemble method for machine learning models. Random forest is a learning model that improves prediction accuracy by using randomly sampled training data and randomly selected explanatory variables to create a plurality of decision trees with low correlation, and integrating and averaging the prediction results. The control parameters of the random forest model include the number of explanatory variables and the number of branches of the decision tree.

Further, as a neural network model, a deep neural network can be mentioned. Compared to machine learning models other than the neural network, the deep neural network has a large number of control parameters of the model and can be flexibly combined, so that the deep neural network can exhibit high performance for various data configurations. Control parameters of the deep neural network include the number of layers of the network, the number of nodes, the type of activation function, the dropout ratio, the mini-batch size, the number of epochs, the learning rate, and the like. There are a plurality of execution frameworks for these models, and the model can be selected from the execution frameworks as appropriate. For example, the execution framework can be selected from Tensorflow, CNTK, Theano, Caffe, mxnet, Keras, PyTorch, Chainer, Scikit-learn, Caret, Matlab (registered trade mark), and the like. In the present embodiment, a neural network is used as a prediction model.

The learning unit 55 learns the neural network as a prediction model and constructs the first learning model M1 and the second learning model M2. First, the first learning model M1 of the first conversion unit 51 will be described. The learning unit 55 learns the neural network using the operating condition parameter as an explanatory variable and the state parameter as an objective variable, and constructs the first learning model M1 by deriving a function representing the relationship between the operating condition parameter and the state parameter. In the present embodiment, the learning unit 55 generates the following functions (1A), (1B), and (1C) that represent the relationship between the operating condition parameter and the state parameter.

$$y1 = w_{u1y1}/[1+\exp\{-(w_{x1u1} \times x1 + w_{x2u1} \times x2 + \ldots + w_{x5u1} \times x5)\}]$$
$$+ w_{u2y1}/[1+\exp\{-(w_{x1u2} \times x1 + w_{x2u2} \times x2 + \ldots + w_{x5u2} \times x5)\}]$$
$$+ w_{u3y1}/[1+\exp\{-(w_{x1u3} \times x1 + w_{x2u3} \times x2 + \ldots + w_{x5u3} \times x5)\}] \ldots (1A)$$

$$y2 = w_{u1y2}/[1+\exp\{-(w_{x1u1} \times x1 + w_{x2u1} \times x2 + \ldots + w_{x5u1} \times x5)\}]$$
$$+ w_{u2y2}/[1+\exp\{-(w_{x1u2} \times x1 + w_{x2u2} \times x2 + \ldots + w_{x5u2} \times x5)\}]$$
$$+ w_{u3y2}/[1+\exp\{-(w_{x1u3} \times x1 + w_{x2u3} \times x2 + \ldots + w_{x5u3} \times x5)\}] \ldots (1B)$$

$$y3 = w_{u1y3}/[1+\exp\{-(w_{x1u1} \times x1 + w_{x2u1} \times x2 + \ldots + w_{x5u1} \times x5)\}]$$
$$+ w_{u2y3}/[1+\exp\{-(w_{x1u2} \times x1 + w_{x2u2} \times x2 + \ldots + w_{x5u2} \times x5)\}]$$
$$+ w_{u3y3}/[1+\exp\{-(w_{x1u3} \times x1 + w_{x2u3} \times x2 + \ldots + w_{x5u3} \times x5)\}] \ldots (1C)$$

In the above functions (1A) to (1C), xi (i is a natural number) is a value of an operating condition parameter, and a maximum value of i is the number of operating condition parameters. In the present embodiment, in a case where five parameters of, for example, the flow rate of the first raw material, the flow rate of the second raw material, the reaction time, the reaction temperature, and the mixing ratio are used as the operating condition parameters, i = 5. ym (m is a natural number) is a value of a state parameter, and a maximum value of m is the number of state parameters. In the present embodiment, in a case where, for example, the color, pressure, and pH of the mixed raw material are used as the state parameters, m = 3. ul (l is a natural number) is a node of a hidden layer L2 in a neural network, which will be described later, and a maximum value of l is the number of nodes. In the present embodiment, l = 3. $w_{xiul}$ and $w_{ulym}$ are weighting coefficients representing the connection weight of the neural network. Specifically, $w_{xiul}$ is a weighting coefficient between xi and ul, and $w_{ulym}$ is a weighting coefficient between ul and ym.

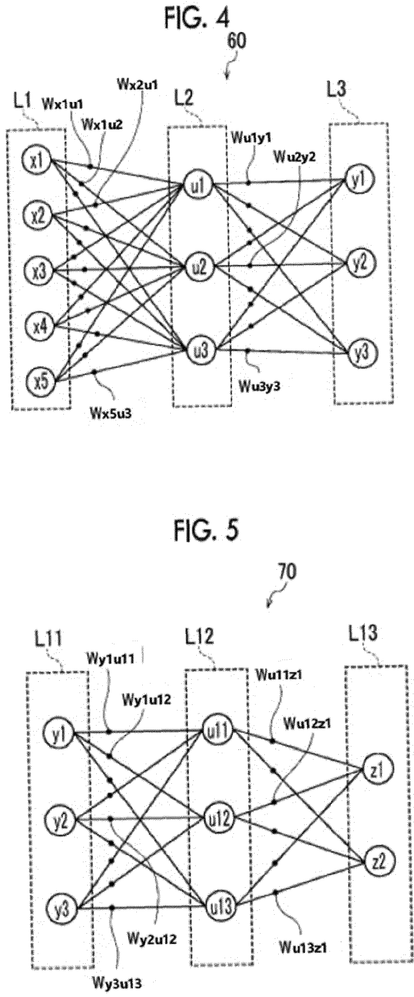

Fig. 4 is a diagram for describing the layer structure of the neural network for constructing the first learning model M1 in the present embodiment. As shown in Fig. 4, a neural network 60 has a three-layer structure of an input layer L1, a hidden layer L2, and an output layer L3. The input layer L1 includes values x1 to x5 of operating condition parameters which are explanatory variables. The hidden layer L2 includes three nodes u1 to u3, and is one layer in the present embodiment. Each of the nodes u1 to u3 is the sum of the values obtained by weighting x1 to x5 with a weighting coefficient $w_{xiul}$ corresponding to each of x1 to x5. The output layer L3 includes values y1 to y3 of state parameters which are objective variables. Each of the values y1 to y3 of the state parameters is a value obtained by weighting nodes u1 to u3 with a weighting coefficient $w_{ulym}$ corresponding to each of the nodes u1 to u3. The black circles "•" in Fig. 4 indicate the weighting coefficients $w_{xiul}$ and $w_{ulym}$. The weighting coefficients $w_{xiul}$ and $w_{ulym}$ are derived by learning the neural network 60 using the teacher data. The layer structure of the neural network 60 is not limited to that shown in Fig. 4.

Next, the second learning model M2 of the second conversion unit 52 will be described. The learning unit 55 learns the neural network using the state parameter as an explanatory variable and the quality parameter as an objective variable, and constructs the second learning model M2 by deriving a function representing the relationship between the state parameter and the quality parameter. In the present embodiment, the learning unit 55 generates the following functions (2A) and (2B) that represent the relationship between the operating condition parameter and the state parameter.

$$] z1 = w_{u1z1}/[1+\exp\{-(w_{y1u11} \times y1 + w_{y2u11} \times y2 + w_{y3u11} \times y3)\}]$$
$$+ w_{u2z1}/[1+\exp\{-(w_{y1u12} \times y1 + w_{y2u12} \times y2 + w_{y3u12} \times y3)\}]$$
$$+ w_{u3z1}/[1+\exp\{-(w_{y1u13} \times y1 + w_{y2u13} \times y2 + w_{y3u13} \times y3)\}] \ldots (2A)$$

$$z2 = w_{u1z2}/[1+\exp\{-(w_{y1u11} \times y1 + w_{y2u11} \times y2 + w_{y3u11} \times y3)\}]$$

$$+ w_{u2z2}/[1+\exp\{-(w_{y1u12} \times y1 + w_{y2u12} \times y2 + w_{y3u12} \times y3)\}]$$

$$+w_{u3z2}/[1+\exp\{-(w_{y1u13} \times y1 + w_{y2u13} \times y2 + w_{y3u13} \times y3)\}] \dots (2B)$$

In the above functions (2A) and (2B), ym (m is a natural number) is a value of a state parameter. In the present embodiment, in a case where, for example, the reaction temperature, color, and pH of the mixed raw material are used as the state parameters as described above, m = 3. zk (k is a natural number) is a value of a quality parameter, and a maximum value of k is the number of quality parameters. In the present embodiment, in a case where, for example, the product concentration and the impurity concentration are used as the quality parameter, k = 2. u1l (l is a natural number) is a node of a hidden layer L12 in a neural network, which will be described later, and a maximum value of 1l is the number of nodes. In the present embodiment, 1l = 3. $w_{ymu1l}$ and $w_{u1lzk}$ are weighting coefficients representing the connection weight of the neural network. Specifically, $w_{ymu1l}$ is a weighting coefficient between ym and u1l, and $w_{u1lzk}$ is a weighting coefficient between u1l and zk.

Fig. 5 is a diagram for describing the layer structure of the neural network for constructing the second learning model M2 in the present embodiment. As shown in Fig. 5, a neural network 70 has a three-layer structure of an input layer L11, a hidden layer L12, and an output layer L13. The input layer L11 includes values y1 to y3 of state parameters which are explanatory variables. The hidden layer L12 includes three nodes u11 to u13, and is one layer in the present embodiment. Each of the nodes u11 to u13 is the sum of the values obtained by weighting y1 to y3 with a weighting coefficient $w_{ymu1l}$ corresponding to each of y1 to y3. The output layer L13 includes values z1 and z2 of quality parameters which are objective variables. Each of the values z1 and z2 of the quality parameters is a value obtained by weighting nodes u1 to u3 with a weighting coefficient $w_{u1lzk}$ corresponding to each of the nodes u1 to u3. The black circles "•" in Fig. 5 indicate the weighting coefficients $w_{ymu1l}$ and $w_{u1lzk}$. The weighting coefficients $w_{ymu1l}$ and $w_{u1lzkym}$ are derived by learning the neural network 70 using the teacher data. The layer structure of the neural network 70 is not limited to that shown in Fig. 5.

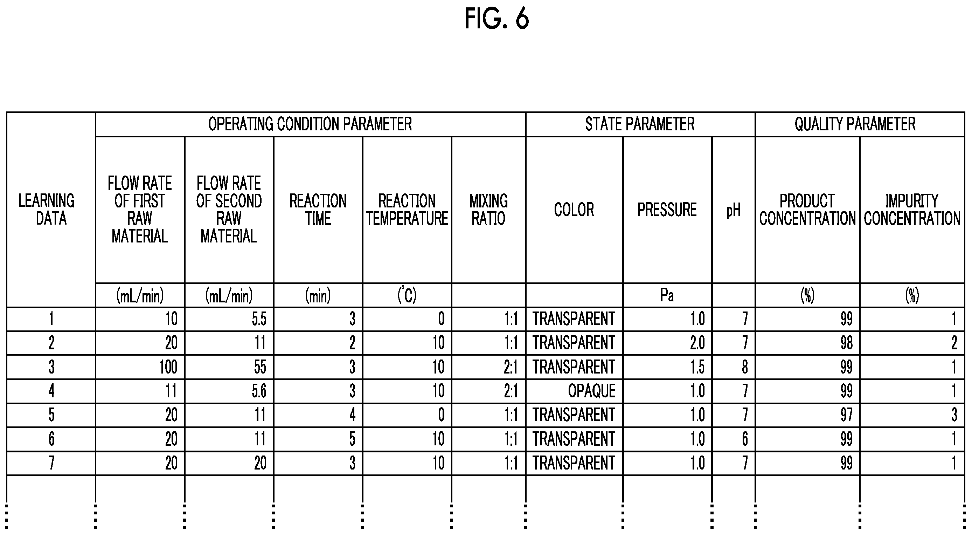

The learning unit 55 learns the neural networks 60 and 70 using a plurality of pieces of teacher data generated in advance to construct the first learning model M1 and the second learning model M2. Here, the teacher data includes operating condition parameters, state parameters, and quality parameters in a case where a product of preferable quality is obtained. A plurality of pieces of teacher data are prepared and stored in the storage 43. Fig. 6 is a diagram showing an example of teacher data. As shown in Fig. 6, the teacher data includes operating condition parameters, state parameters, and quality parameters. Further, the operating condition parameters include five parameters of the flow rate of the first raw material, the flow rate of the second raw material, the reaction time, the reaction temperature, and the mixing ratio. The state parameters include three parameters of color, pressure, and pH of the mixed raw material. The quality parameters also include two parameters of product concentration and impurity concentration.

At the time of learning, the learning unit 55 trains the neural networks 60 and 70 using the teacher data stored in the storage 43, for example, according to the error back propagation method. Specifically, for the neural network 60, the learning unit 55 inputs the operating condition parameter included in one of the teacher data sets to the neural network 60, and causes the neural network 60 to output the state parameter. Then, the learning unit 55 trains the neural network 60 by deriving the weighting coefficients $w_{xiul}$ and $w_{ulym}$ so that the difference between the state parameter output from the neural network 60 and the state parameter included in the teacher data is minimized.

Further, for the neural network 70, the learning unit 55 inputs the state parameter included in one of the teacher data sets to the neural network 70, and causes the neural network 70 to output the quality parameter. Then, the learning unit 55 trains the neural network 70 by deriving the weighting coefficients $w_{ymu1l}$ and $w_{u1lzk}$ so that the difference between the quality parameter output from the neural network 70 and the quality parameter included in the teacher data is minimized.

In a case where the learning is completed and the first learning model M1 and the second learning model M2 are constructed, the learning unit 55 stores the functions represented by the first learning model M1 and the second learning model M2 in the storage 43.

In the present embodiment, the third conversion unit 53 and the fourth conversion unit 54 derive operating condition parameters for obtaining target quality parameters from target unknown quality parameters (hereinafter referred to as target quality parameters). For this purpose, the third conversion unit 53 converts the target quality parameter into a state parameter based on the control parameter of the second learning model M2 of the second conversion unit 52. The state parameter obtained by converting the target quality parameter is referred to as a target state parameter. The control parameters are the weighting coefficients $w_{ymu1l}$ and $w_{u1lzk}$ in the function obtained based on the second learning model M2. In the present embodiment, the third conversion unit 53 derives the following functions G21 and G22 for generating the target state parameter.

$$G21 = zt1-[w_{u1z1}/[1+\exp\{-(w_{y1u11} \times y1 + w_{y2u11} \times y2 + w_{y3u11} \times y3)\}] +$$

$$w_{u2z1}/[1+\exp\{-(w_{y1u12} \times y1 + w_{y2u12} \times y2 + w_{y3u12} \times y3)\}]$$

$$+ w_{u3z1}/[1+\exp\{-(w_{y1u13} \times y1 + w_{y2u13} \times y2 + w_{y3u13} \times y3)\}]] \dots (3A)$$

$$G22 = zt2-[w_{u1z2}/[1+\exp\{-(w_{y1u11} \times y1 + w_{y2u11} \times y2 + w_{y3u11} \times y3)\}] +$$

$$w_{u2z2}/[1+\exp\{-(w_{y1u12} \times y1 + w_{y2u12} \times y2 + w_{y3u12} \times y3)\}]$$

$$+w_{u3z2}/[1+\exp\{-(w_{y1u13} \times y1 + w_{y2u13} \times y2 + w_{y3u13} \times y3)\}]] \dots (3B)$$

In the functions G21 and G22, zt1 and zt2 are target quality parameters, respectively. The third conversion unit 53 derives the state parameter ym for minimizing the absolute value of the functions G21 and G22 as a target state parameter ytm. Specifically, the target state parameter ytm is derived by performing multivariate analysis such as multiple regression analysis and principal component analysis, or multi-objective optimization such as genetic algorithm, multi-objective particle swarm optimization, and Bayesian optimization using the target quality parameters zt1 and zt2 as explanatory variables and the target state parameter ytm as the objective variable.

The fourth conversion unit 54 converts the target state parameter into an operating condition parameter based on the control parameter of the first learning model M1 of the first conversion unit 51. The operating condition parameter obtained by converting the target state parameter is used as a target operating condition parameter. The control parameters are the weighting coefficients $w_{xiul}$ and $w_{ulym}$ in the function represented by the first learning model M1. In the present embodiment, the fourth conversion unit 54 generates the following functions G11, G12, and G13 for generating the target operating condition parameter.

$$G11 = yt1-[w_{u1y1}/[1+\exp\{-(w_{x1u1} \times x1 + w_{x2u1} \times x2 + \dots + w_{x5u1} \times x5)\}]$$

$$+ w_{u2y1}/[1+\exp\{-(w_{x1u2} \times x1 + w_{x2u2} \times x2 + \dots + w_{x5u2} \times x5)\}]$$

$$+ w_{u3y1}/[1+\exp\{-(w_{x1u3} \times x1 + w_{x2u3} \times x2 + \dots + w_{x5u3} \times x5)\}]] \dots (4A)$$

$$G12 = yt2-[w_{u1y2}/[1 + \exp\{-(w_{x1u1} \times x1 + w_{x2u1} \times x2 + \dots + w_{x5u1} \times x5)\}]$$

$$+ w_{u2y2}/[1 + \exp\{-(w_{x1u2} \times x1 + w_{x2u2} \times x2 + \dots + w_{x5u2} \times x5)\}]$$

$$+ w_{u3y2}/[1+\exp\{-(w_{x1u3} \times x1 + w_{x2u3} \times x2 + \dots + w_{x5u3} \times x5)\}]] \dots (4B)$$

$$G13 = yt3-[w_{u1y3}/[1+\exp\{-(w_{x1u1} \times x1 + w_{x2u1} \times x2 + \dots + w_{x5u1} \times x5)\}]$$

$$+ w_{u2y3}/[1+\exp\{-(w_{x1u2} \times x1 + w_{x2u2} \times x2 + \dots + w_{x5u2} \times x5)\}]$$

$$+ w_{u3y3}/[1+\exp\{-(w_{x1u3} \times x1 + w_{x2u3} \times x2 + \dots + w_{x5u3} \times x5)\}]] \dots (4C)$$

In the functions G11 to G13, yt1, yt2, and yt3 are target state parameters, respectively. The fourth conversion unit 54 derives the operating condition parameter xi for minimizing the absolute value of the functions G11 to G13 as a target operating condition parameter xti. Specifically, the target operating condition parameter xti is derived by performing multivariate analysis such as multiple regression analysis and principal component analysis, or multi-objective optimization such as genetic algorithm, multi-objective particle swarm optimization, and Bayesian optimization using the target state parameters yt1, yt2, and yt3 as explanatory variables and the target operating condition parameter xti as the objective variable.

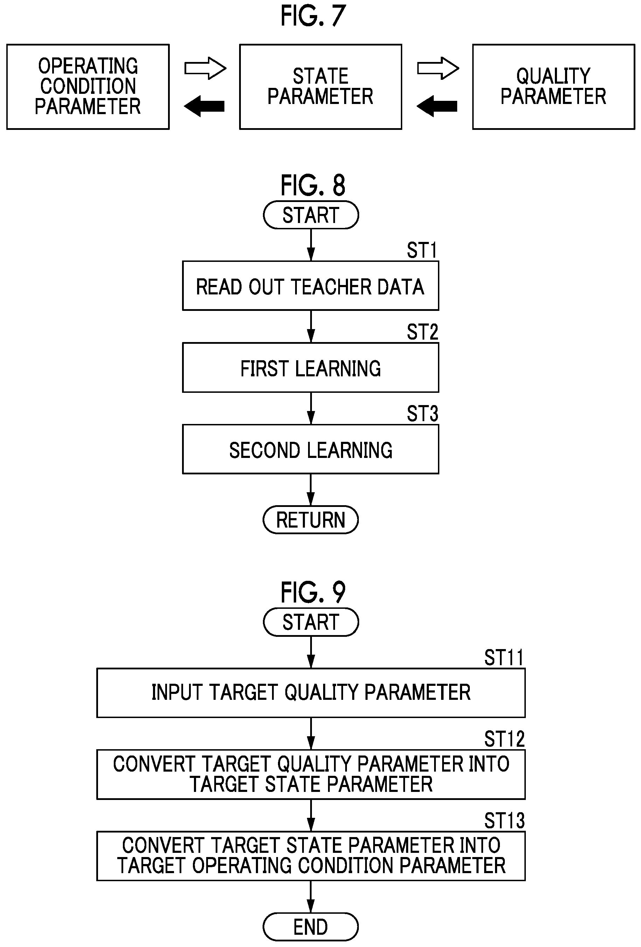

The processing performed by the first conversion unit 51, the second conversion unit 52, the third conversion unit 53, and the fourth conversion unit 54 described above will be conceptually described with reference to Fig. 7. The first conversion unit 51 predicts the state parameter as a forward problem from the operating condition parameter. The second conversion unit 52 predicts the quality parameter as a forward problem from the state parameter. The third conversion unit 53 predicts the state parameter as an inverse problem from the quality parameter. The fourth conversion unit 54 predicts the operating condition parameter as an inverse problem from the state parameter. In Fig. 7, the white arrow represents the forward problem, and the black arrow represents the inverse problem.

As shown in Fig. 7, in the present embodiment, the quality parameter is derived from the operating condition parameter by the two-step process through the state parameter. Further, the operating condition parameter is derived by the two-step process from the quality parameter to the state parameter.

The optimization support device 3 outputs the target operating condition parameter xti to the controller 12. The controller 12 controls the operation of the flow reactor 11 according to the target operating condition parameter xti. Accordingly, a product having the target quality is produced.

Next, a process performed in the present embodiment will be described. Fig. 8 is a flowchart showing a process performed at the time of constructing the first learning model M1 of the first conversion unit 51 and the second learning model M2 of the second conversion unit 52 in the present embodiment. The learning unit 55 reads out one piece of teacher data from a plurality of pieces of teacher data stored in the storage 43 (Step ST1), and causes the neural network of the first conversion unit 51 to learn the relationship between the operating condition parameter and the state parameter (first learning; Step ST2). Next, the learning unit 55 causes the neural network of the second conversion unit 52 to learn the relationship between the state parameter and the quality parameter (second learning; Step ST3). Then, the learning unit returns to Step ST1, reads out the next teacher data, and repeats the processes of Step ST2 and Step ST3. Thereby, the first learning model M1 and the second learning model M2 are constructed.

The learning unit 55 repeats learning until a difference between the state parameter generated by the first conversion unit 51 and the state parameter of the teacher data and a difference between the quality parameter generated by the second conversion unit 52 and the quality parameter of the teacher data are equal to or less than a predetermined threshold value. The number of repetitions may be a predetermined number of times.

Next, in the present embodiment, the process for deriving the operating condition parameter from the unknown target quality parameter will be described. Fig. 9 is a flowchart showing a process for deriving the operating condition parameter from the target quality parameter. In a case where the target quality parameter is input to the optimization support device 3 from the input unit 15 (Step ST11), the third conversion unit 53 converts the target quality parameter into the target state parameter based on the control parameter of the second learning model M2 (Step ST12). Next, the fourth conversion unit 54 converts the target state parameter into the target operating condition parameter (Step ST13), and ends the process.

As described above, a quality parameter was derived from a process condition parameter in the related art, but in the present embodiment, a process condition parameter is divided into an operating condition parameter and a state parameter, and a quality parameter is derived by a two-step process of converting an operating condition parameter into a state parameter by the first conversion unit 51 and converting the state parameter into the quality parameter by the second conversion unit 52. Therefore, compared with the process of obtaining quality parameters by using operating condition parameters and state parameters as process condition parameters without distinguishing between the operating condition parameters and the state parameters in the related art, the number of input parameters can be reduced in the processes performed by the first conversion unit 51 and the second conversion unit 52, respectively. Thereby, overtraining can be prevented, especially in a case where a learning model constructed by learning a neural network or the like is used for conversion. Therefore, according to the present embodiment, various conditions in the product production process can be accurately predicted.

Further, in the present embodiment, the process of deriving the target operating condition parameter from the target quality parameter is performed in each of the third conversion unit 53 and the fourth conversion unit 54, so that the process is divided into two processes. Therefore, compared with the process of obtaining process condition parameters from quality parameters without distinguishing between the operating condition parameters and the state parameters in the related art, the number of input parameters can be reduced in the processes performed by the third conversion unit 53 and the fourth conversion unit 54, respectively. Thereby, according to the present embodiment, the target operating condition parameter can be accurately predicted from the target quality parameter.

Further, in the present embodiment, the conversion of the target quality parameter to the target state parameter is performed using the control parameter of the second learning model M2 and the conversion from the target state parameter to the target operating condition parameter is performed using the control parameter of the first learning model M1. Here, the control parameters of the first learning model M1 and the second learning model M2 can accurately predict various conditions in the product production process. Therefore, according to the present embodiment, the target operating condition parameter can be accurately derived from the target quality parameter. Therefore, by producing the product using the target operating condition parameter, the product having the target quality can be obtained.

Further, in a case where the process condition parameter is predicted from the quality parameter by learning the correlation between the process condition parameter and the quality parameter as in the method described in JP2003-345416A, the correlation between the operating condition parameter and the state parameter is not learned at all as in the present embodiment. Therefore, even though the production process is performed according to the predicted process condition parameters, the product having the target quality may not be produced. That is, in a case where the process condition parameter is predicted from the quality parameter by learning the correlation between the process condition parameter and the quality parameter, it is assumed that the state parameter can be realized by the operating condition parameter. However, that assumption is not established in most cases. Therefore, it is not possible to accurately predict the process condition parameter that can achieve the target quality parameter by the method of predicting the process condition parameter from the quality parameter as in the related art.

In the present embodiment, a process condition parameter is divided into an operating condition parameter and a state parameter, and a state parameter is predicted from a quality parameter and an operating condition parameter is predicted from the state parameter, or a state parameter is predicted from an operating condition parameter and a quality parameter is predicted from the state parameter. Therefore, the operating condition parameter, the state parameter, and the quality parameter can be predicted in the situation where the assumption that the state parameter can be realized by the operating condition parameter included in the process condition parameter is established. Therefore, according to the present embodiment, various conditions in the product production process can be accurately predicted.

In the above embodiment, the first conversion unit 51 converts the operating condition parameter into the state parameter by using the first learning model M1 constructed by learning the neural network 60, but the present disclosure is not limited thereto. For example, the operating condition parameter may be converted into the state parameter by a mathematical formula for converting the operating condition parameter into the state parameter, a table in which the operating condition parameter and the state parameter are associated with each other, or the like.

In this case, the fourth conversion unit 54 may convert the state parameter into the operating condition parameter by a mathematical formula for converting the state parameter into the operating condition parameter, a table in which the state parameter and the operating condition parameter are associated with each other, or the like.

Further, in the above embodiment, the second conversion unit 52 converts the state parameter into the quality parameter by using the second learning model M2 constructed by learning the neural network 70, but the present disclosure is not limited thereto. For example, the state parameter may be converted into the quality parameter by a mathematical formula for converting the state parameter into the quality parameter, a table in which the state parameter and the quality parameter are associated with each other, or the like.

In this case, the third conversion unit 53 may convert the quality parameter into the state parameter by a mathematical formula for converting the quality parameter into the state parameter, a table in which the quality parameter and the state parameter are associated with each other, or the like.

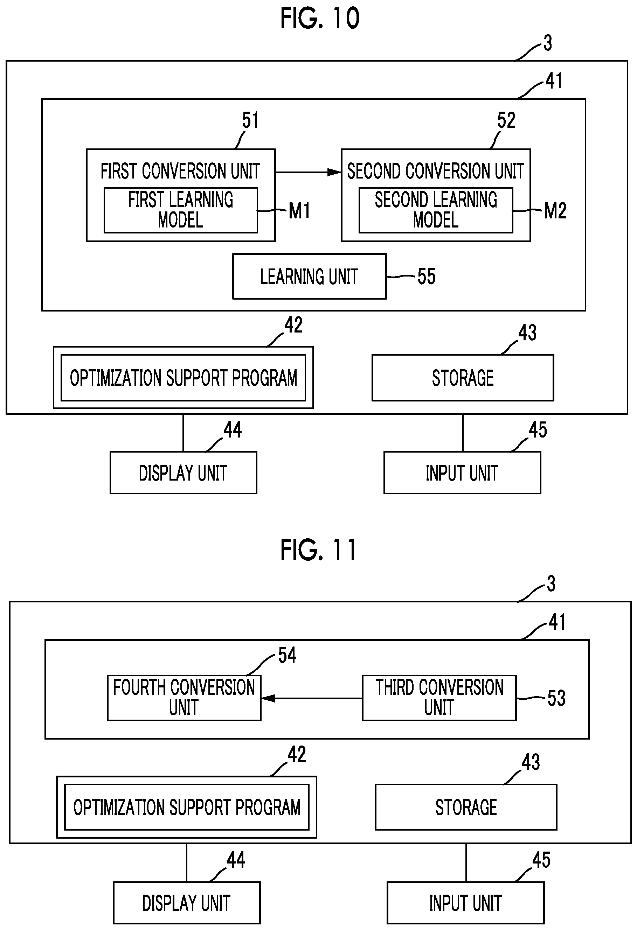

Further, in the above embodiment, the optimization support device 3 comprises the first conversion unit 51, the second conversion unit 52, the third conversion unit 53, and the fourth conversion unit 54, but the present disclosure is not limited thereto. As shown in Fig. 10, the optimization support device 3 may have only the first conversion unit 51 and the second conversion unit 52. In this case, the first conversion unit 51 may convert the operating condition parameter into the state parameter by the first learning model M1, or may convert the operating condition parameter into the state parameter by a mathematical formula, a table, or the like. Further, the second conversion unit 52 may convert the state parameter into the quality parameter by the second learning model M2, or may convert the state parameter into the quality parameter by a mathematical formula, a table, or the like.

Further, as shown in Fig. 11, the optimization support device 3 may have only the third conversion unit 53 and the fourth conversion unit 54. In this case, the third conversion unit 53 may convert the quality parameter into the state parameter by using the control parameter of the second learning model M2 constructed by a device separate from the optimization support device 3, or may convert the quality parameter into the state parameter by a mathematical formula, a table, or the like. Further, the fourth conversion unit 54 may convert the state parameter into the operating condition parameter by using the control parameter of the first learning model M1 constructed by a device separate from the optimization support device 3, or may convert the state parameter into the operating condition parameter by a mathematical formula, a table, or the like.

Further, in the above embodiment, the flow synthesis process is used as the process for producing the product, but the present disclosure is not limited thereto. A cell culture process may be used as the process for producing the product. In the cell culture process, the product is a cell and an antibody produced by the cell, the cell culture condition is used as an operating condition parameter, and the quality of the cell and the antibody produced by the cell is used as a quality parameter. Further, the production device 2 shown in Fig. 1 is a cell culture device. For example, in a case where the cell culture process is an antibody cell culture process, as the operating condition parameter, for example, a perfusion ratio, the number of cells in a stationary phase, a stirring speed of a liquid in a culture tank, the amount of bottom air, the amount of bottom oxygen, the amount of bottom nitrogen, the amount of carbon dioxide on the upper surface, the amount of air on the upper surface, the amount of antifoaming agent, the amount of surfactant, and the like can be used. As the state parameter, for example, pH, $pO_2$ (oxygen partial pressure), $pCO_2$ (carbon dioxide partial pressure), Gln (glutamine concentration), Glu (glutamic acid concentration), Gluc (glucose concentration), Lac (lactic acid concentration), $NH_4^+$ (ammonia ion concentration), $Na^+$ (sodium ion concentration), $K^+$ (potassium ion concentration), Osmol (osmotic pressure), Kla (oxygen transfer coefficient), and the like can be used. As the quality parameter, antibody concentration, by-product concentration, the number of living cells, and the like can be used.

Further, a vacuum film forming process may be used as the process for producing the product. In the vacuum film forming process, the product is a film formed on the surface of a substrate such as glass, resin, and metal. Further, the production device 2 shown in Fig. 1 is a vacuum film forming device. Further, the condition of vacuum film formation is used as an operating condition parameter, and the quality of film formation is used as a quality parameter. As the operating condition parameter, for example, vacuum pressure, applied voltage, bias voltage, substrate temperature, film formation time, gas flow rate, gas concentration, line speed, and the like can be used. The state parameter is the state of plasma, and plasma stability, plasma color development, ICP-OES/ICP-AES (plasma emission spectrometry) waveform spectrum, plasma density, adhesive plate depot thickness, device potential difference, and the like can be used. As the quality parameter, film quality, barrier performance, and the like can be used.

A roll-to-roll coating process may also be used as the process for producing the product. In the coating process, the product is a liquid crystal retardation film, an antiglare film, or the like in which a coating film is formed on the surface of a resin serving as a substrate, such as triacetyl cellulose (TAC), polyethylene terephthalate (PET), and cycloolefin polymer (COP). Further, the production device 2 shown in Fig. 1 is a coating device. Further, the coating condition is used as an operating condition parameter, and the quality of the coating film is used as a quality parameter. As the operating condition parameter, for example, the coating flow rate, the liquid temperature, the drying temperature, the line speed, the transport roll temperature, and the like can be used. As the state parameter, the pulsation of the liquid, the fluctuation of the drying air velocity, the amount of volatilization during drying, the amount of liquid permeation into the substrate, and the like can be used. As the quality parameter, the film quality, the film thickness distribution of the coating film, the adhesion between the coating film and the substrate, and the like can be used.

In the above embodiments, for example, as hardware structures of processing units that execute various kinds of processing, such as the first conversion unit 51, the second conversion unit 52, the third conversion unit 53, the fourth conversion unit 54, and the learning unit 55, various processors shown below can be used. As described above, the various processors include a programmable logic device (PLD) as a processor of which the circuit configuration can be changed after manufacture, such as a field programmable gate array (FPGA), a dedicated electrical circuit as a processor having a dedicated circuit configuration for executing specific processing such as an application specific integrated circuit (ASIC), and the like, in addition to the CPU as a general-purpose processor that functions as various processing units by executing software (program).

One processing unit may be configured by one of the various processors, or configured by a combination of the same or different kinds of two or more processors (for example, a combination of a plurality of FPGAs or a combination of the CPU and the FPGA). In addition, a plurality of processing units may be configured by one processor.

As an example where a plurality of processing units are configured by one processor, first, there is a form in which one processor is configured by a combination of one or more CPUs and software as typified by a computer, such as a client or a server, and this processor functions as a plurality of processing units. Second, there is a form in which a processor for realizing the function of the entire system including a plurality of processing units by one integrated circuit (IC) chip as typified by a system on chip (SoC) or the like is used. In this way, various processing units are configured by one or more of the above-described various processors as hardware structures.

Furthermore, as the hardware structure of the various processors, more specifically, an electrical circuit (circuitry) in which circuit elements such as semiconductor elements are combined can be used.

Explanation of References

1: production facility
2: production device
3: optimization support device
11: flow reactor
12: controller
21: first supply unit
22: second supply unit
23: reaction section
26: collecting section
31: merging portion
31a to 31c: first tube part to third tube part
32: reaction portion
33: temperature control unit
34: irradiation unit
35: first detection unit
36: second detection unit
41: CPU
42: memory

43: storage
44: display unit
45: input unit
51: first conversion unit
52: second conversion unit
53: third conversion unit
54: fourth conversion unit
55: learning unit
60, 70: neural network
L1, L11: input layer
L2, L12: hidden layer
L3, L13: output layer
xi: value of operating condition parameter
ul, u1l: node value
ym: value of state parameter
zk: value of quality parameter

**Claims**

1. A production facility (1) comprises a production device (2) and a support device (3),

wherein the production device (2) comprises a flow reactor (11) and a controller (12),
wherein the support device (3) is configured to determine a target operating condition parameter for a flow reaction process performed by the flow reactor (11),
wherein the flow reactor (11) comprises a first supply unit (21), a second supply unit (22), a reaction section (23), and a collecting section (26), the operation of each part of the flow reactor (11) is controlled by the controller (12), and the controller (12) is connected to the support device (3),
wherein in the flow reactor (11), the first supply unit (21) and the second supply unit (22) are respectively connected to upstream end parts of the reaction section (23) by piping, and the collecting section (26) is connected to a downstream end part of the reaction section (23) by piping,
wherein the first supply unit (21) is configured to supply a first raw material of the flow reaction to the reaction section (23),
wherein the second supply unit (22) is configured to supply a second raw material of the flow reaction to the reaction section (23),
wherein the reaction section (23) comprises a first detection unit (35) configured to detect the state of the mixed raw material in the reaction section (23) and output the result of the state of the mixed raw material to the support device (3) as teacher data,
wherein the collecting section (26) comprises a second detection unit (36) configured to detect the quality of the product, which is the processing result of the flow reaction, and outputs the detected result to the support device (3) as teacher data,
wherein the support device (3) comprises:

a first conversion unit (51) configured to convert an operating condition parameter indicating an operating condition of the flow reaction process into a state parameter, the operating condition parameter being a parameter for setting a reaction condition of the flow reaction process and for driving each part of the flow reactor (11), the state parameter being a parameter indicating physical properties of mixed raw material obtained when the mixed raw material is reacted; and
a second conversion unit (52) configured to convert the state parameter into a quality parameter indicating a quality of the product,
wherein the first conversion unit (51) has a first learning model that has been trained using the result of the state of the mixed raw material to output the state parameter by inputting the operating condition parameter, and
the second conversion unit (52) has a second learning model that has been trained using the processing result of the flow reaction to output the quality parameter by inputting the state parameter,
wherein the support device further comprises:

a third conversion unit (53) configured to convert a target quality parameter into a state parameter based on a control parameter of the second learning model that has been trained to output the quality parameter by inputting the state parameter; and

a fourth conversion unit (54) configured to convert the state parameter into the target operating condition parameter for obtaining the target quality parameter based on a control parameter of the first learning model that has been trained to output the state parameter by inputting the operating condition parameter, wherein the support device (3) is configured to output the target operating condition parameter to the controller (12), and

wherein the controller (12) is configured to control the operation of the flow reactor (11) according to the target operating condition parameter, whereby a product having the target quality is produced.

## Patentansprüche

1. Produktionsanlage (1) umfasst eine Produktionsvorrichtung (2) und eine Unterstützungsvorrichtung (3),

wobei die Produktionsvorrichtung (2) einen Strömungsreaktor (11) und eine Steuerung (12) umfasst,

wobei die Unterstützungsvorrichtung (3) so konfiguriert ist, dass sie einen Zielbetriebsbedingungsparameter für einen Strömungsreaktionsprozess, der von dem Strömungsreaktor (11) durchgeführt wird, bestimmt,

wobei der Strömungsreaktor (11) eine erste Zufuhreinheit (21), eine zweite Zufuhreinheit (22), einen Reaktionsabschnitt (23) und einen Sammelabschnitt (26) umfasst, der Betrieb jedes Teils des Strömungsreaktors (11) von der Steuerung (12) gesteuert wird und die Steuerung (12) mit der Unterstützungsvorrichtung (3) verbunden ist,

wobei in dem Strömungsreaktor (11) die erste Zufuhreinheit (21) und die zweite Zufuhreinheit (22) entsprechend durch Rohrleitungen mit stromaufwärtigen Endteilen des Reaktionsabschnitts (23) verbunden sind und der Sammelabschnitt (26) durch Rohrleitung mit einem stromabwärtigen Endteil des Reaktionsabschnitts (23) verbunden ist,

wobei die erste Zufuhreinheit (21) so konfiguriert ist, dass sie dem Reaktionsabschnitt (23) einen ersten Rohstoff der Strömungsreaktion zuführt,

wobei die zweite Zufuhreinheit (22) so konfiguriert ist, dass sie dem Reaktionsabschnitt (23) einen zweiten Rohstoff der Strömungsreaktion zuführt,

wobei der Reaktionsabschnitt (23) eine erste Detektionseinheit (35) umfasst, die so konfiguriert ist, dass sie den Zustand des gemischten Rohstoffs in dem Reaktionsabschnitt (23) detektiert und das Ergebnis des Zustands des gemischten Rohstoffs als Lehrerdaten an die Unterstützungsvorrichtung (3) ausgibt,

wobei der Sammelabschnitt (26) eine zweite Detektionseinheit (36) umfasst, die so konfiguriert ist, dass sie die Qualität des Produkts, das das Verarbeitungsergebnis der Strömungsreaktion ist, detektiert und das detektierte Ergebnis als Lehrerdaten an die Unterstützungsvorrichtung (3) ausgibt,

wobei die Unterstützungsvorrichtung (3) umfasst:

eine erste Umwandlungseinheit (51), die so konfiguriert ist, dass sie einen Betriebsbedingungenparameter, der eine Betriebsbedingung des Strömungsreaktionsprozesses angibt, in einen Zustandsparameter umwandelt, wobei der Betriebsbedingungenparameter ein Parameter zum Einstellen einer Reaktionsbedingung des Strömungsreaktionsprozesses und zum Antreiben jedes Teils des Strömungsreaktors (11) ist und der Zustandsparameter ein Parameter ist, der physikalische Eigenschaften von gemischtem Rohstoff, der erhalten wird, wenn der gemischte Rohstoff umgesetzt wird, angibt; und

eine zweite Umwandlungseinheit (52), die so konfiguriert ist, dass sie den Zustandsparameter in einen Qualitätsparameter, der eine Qualität des Produkts angibt, umwandelt,

wobei die erste Umwandlungseinheit (51) ein erstes Lernmodell aufweist, das unter Verwendung des Ergebnisses des Zustands des gemischten Rohstoffs trainiert wurde, um den Zustandsparameter durch Eingeben des Betriebsbedingungenparameters auszugeben, und

die zweite Umwandlungseinheit (52) ein zweites Lernmodell aufweist, das unter Verwendung des Verarbeitungsergebnisses der Strömungsreaktion trainiert wurde, um den Qualitätsparameter durch Eingeben des Zustandsparameters auszugeben,

wobei die Unterstützungsvorrichtung ferner umfasst:

eine dritte Umwandlungseinheit (53), die so konfiguriert ist, dass sie einen Zielqualitätsparameter in einen Zustandsparameter auf der Grundlage eines Steuerparameters des zweiten Lernmodells, das trainiert wurde, um den Qualitätsparameter durch Eingeben des Zustandsparameters auszugeben, umwandelt; und

eine vierte Umwandlungseinheit (54), die so konfiguriert ist, dass sie den Zustandsparameter in den Zielbetriebsbedingungsparameter zum Erhalten des Zielqualitätsparameters auf der Grundlage eines Steuerparameters des ersten Lernmodells, das trainiert wurde, um den Zustandsparameter durch Eingeben des Betriebsbedingungsparameters auszugeben, umwandelt,

wobei die Unterstützungsvorrichtung (3) so konfiguriert ist, dass sie den Zielbetriebsbedingungsparameter an die Steuerung (12) ausgibt, und

wobei die Steuerung (12) so konfiguriert ist, dass sie den Betrieb des Strömungsreaktors (11) gemäß dem Zielbetriebsbedingungsparameter steuert, wodurch ein Produkt mit der Zielqualität hergestellt wird.

## Revendications

1. Installation de production (1) comprenant un dispositif de production (2) et un dispositif de support (3),

dans laquelle le dispositif de production (2) comprend un réacteur d'écoulement (11) et un contrôleur (12),

dans laquelle le dispositif de support (3) est configuré pour déterminer un paramètre de condition d'opération cible pour un processus de réaction d'écoulement effectué par le réacteur d'écoulement (11),

dans laquelle le réacteur d'écoulement (11) comprend une première unité d'alimentation (21), une deuxième unité d'alimentation (22), une section de réaction (23) et une section de collecte (26), le fonctionnement de chaque partie du réacteur d'écoulement (11) est contrôlé par le contrôleur (12), et le contrôleur (12) est connecté au dispositif de support (3),

dans laquelle dans le réacteur d'écoulement (11), la première unité d'alimentation (21) et la deuxième unité d'alimentation (22) sont respectivement connectées à des parties d'extrémité en amont de la section de réaction (23) par tuyauterie, et la section de collecte (26) est connectée à une partie d'extrémité en aval de la section de réaction (23) par tuyauterie,

dans laquelle la première unité d'alimentation (21) est configurée pour fournir une première matière première de la réaction d'écoulement à la section de réaction (23), dans laquelle la deuxième unité d'alimentation (22) est configurée pour fournir une deuxième matière première de la réaction d'écoulement à la section de réaction (23),

dans laquelle la section de réaction (23) comprend une première unité de détection (35) configurée pour détecter l'état de la matière première mélangée dans la section de réaction (23) et sortir le résultat de l'état de la matière première mélangée au dispositif de support (3) comme des données d'enseignant,

dans laquelle la section de collecte (26) comprend une deuxième unité de détection (36) configurée pour détecter la qualité du produit, qui est le résultat de traitement de la réaction d'écoulement, et sort le résultat détecté au dispositif de support (3) comme des données d'enseignant,

dans laquelle le dispositif de support (3) comprend :

une première unité de conversion (51) configurée pour convertir un paramètre de condition d'opération indiquant une condition d'opération du processus de réaction d'écoulement en un paramètre d'état, le paramètre de condition d'opération étant un paramètre pour régler une condition de réaction du processus de réaction d'écoulement et pour entraîner chaque partie du réacteur d'écoulement (11), le paramètre d'état étant un paramètre indiquant des propriétés physiques de matière première mélangée obtenue lorsque la matière première mélangée est mise en réaction ; et

une deuxième unité de conversion (52) configurée pour convertir le paramètre d'état en un paramètre de qualité indiquant une qualité du produit,

dans laquelle la première unité de conversion (51) a un premier modèle d'apprentissage qui a été entraîné en utilisant le résultat de l'état de la matière première mélangée pour sortir le paramètre d'état en entrant le paramètre de condition d'opération, et

la deuxième unité de conversion (52) a un deuxième modèle d'apprentissage qui a été entraîné en utilisant le résultat de traitement de la réaction d'écoulement pour sortir le paramètre de qualité en entrant le paramètre d'état,

dans laquelle le dispositif de support comprend en outre :

une troisième unité de conversion (53) configurée pour convertir un paramètre de qualité cible en un paramètre d'état sur la base d'un paramètre de contrôle du deuxième modèle d'apprentissage qui a été entraîné pour sortir le paramètre de qualité en entrant le paramètre d'état ; et

une quatrième unité de conversion (54) configurée pour convertir le paramètre d'état en le paramètre de condition d'opération cible pour obtenir le paramètre de qualité cible sur la base d'un paramètre de contrôle du premier modèle d'apprentissage qui a été entraîné pour sortir le paramètre d'état en entrant le paramètre de condition d'opération,

dans laquelle le dispositif de support (3) est configuré pour sortir le paramètre de condition d'opération cible au contrôleur (12), et

dans laquelle le contrôleur (12) est configuré pour contrôler le fonctionnement du réacteur d'écoulement (11) en fonction du paramètre de condition d'opération cible, de sorte qu'un produit ayant la qualité cible est produit.

# FIG. 1

1

2

PRODUCTION DEVICE

3

OPTIMIZATION
SUPPORT DEVICE

# FIG. 2

1

2

11

FIRST SUPPLY
UNIT

21

31

31a  31c  23

32

26

COLLECTING
SECTION

31b

22

SECOND SUPPLY
UNIT

IRRADIATION
UNIT

TEMPERATURE
CONTROL UNIT

FIRST
DETECTION
UNIT

SECOND
DETECTION
UNIT

34  33  35  36

12

CONTROLLER

OPTIMIZATION
SUPPORT DEVICE

3

# FIG. 3

# FIG. 4

# FIG. 5

## FIG. 6

| LEARNING DATA | OPERATING CONDITION PARAMETER | | | | | STATE PARAMETER | | | QUALITY PARAMETER | |
|---|---|---|---|---|---|---|---|---|---|---|
| | FLOW RATE OF FIRST RAW MATERIAL | FLOW RATE OF SECOND RAW MATERIAL | REACTION TIME | REACTION TEMPERATURE | MIXING RATIO | COLOR | PRESSURE | pH | PRODUCT CONCENTRATION | IMPURITY CONCENTRATION |
| | (mL/min) | (mL/min) | (min) | (°C) | | | Pa | | (%) | (%) |
| 1 | 10 | 5.5 | 3 | 0 | 1:1 | TRANSPARENT | 1.0 | 7 | 99 | 1 |
| 2 | 20 | 11 | 2 | 10 | 1:1 | TRANSPARENT | 2.0 | 7 | 98 | 2 |
| 3 | 100 | 55 | 3 | 10 | 2:1 | TRANSPARENT | 1.5 | 8 | 99 | 1 |
| 4 | 11 | 5.6 | 3 | 10 | 2:1 | OPAQUE | 1.0 | 7 | 99 | 1 |
| 5 | 20 | 11 | 4 | 0 | 1:1 | TRANSPARENT | 1.0 | 7 | 97 | 3 |
| 6 | 20 | 11 | 5 | 10 | 1:1 | TRANSPARENT | 1.0 | 6 | 99 | 1 |
| 7 | 20 | 20 | 3 | 10 | 1:1 | TRANSPARENT | 1.0 | 7 | 99 | 1 |
| ⋮ | ⋮ | ⋮ | ⋮ | ⋮ | ⋮ | ⋮ | ⋮ | ⋮ | ⋮ | ⋮ |

EP 3 995 917 B1

## FIG. 7

| OPERATING CONDITION PARAMETER | ⇒ ← | STATE PARAMETER | ⇒ ← | QUALITY PARAMETER |

## FIG. 8

START

ST1
READ OUT TEACHER DATA

ST2
FIRST LEARNING

ST3
SECOND LEARNING

RETURN

## FIG. 9

START

ST11
INPUT TARGET QUALITY PARAMETER

ST12
CONVERT TARGET QUALITY PARAMETER INTO TARGET STATE PARAMETER

ST13
CONVERT TARGET STATE PARAMETER INTO TARGET OPERATING CONDITION PARAMETER

END

# FIG. 10

# FIG. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003345416 A
- JP 2006323523 A
- JP H06304724 A, NIPPON STEEL CORP
- WO 2018072773 A2
- WO 9900717 A1, HONEYWELL INC